# EUROPEAN PATENT SPECIFICATION

(11) **EP 3 709 954 B1**
(45) Date of publication and mention of the grant of the patent: **26.02.2025**
(21) Application number: 18800090.5
(22) Date of filing: 07.11.2018
(51) Int. Cl.: A61K 8/34, A61K 8/35, A61Q 17/00, A61K 31/04, A61K 31/12, A61P 17/10, A61K 9/00, A61K 9/06, A61K 9/10

(54) **TOPICAL COMPOSITIONS OF PHYTANTRIOL AND P-HYDROXYACETOPHENONE**
TOPISCHE ZUSAMMENSETZUNGEN VON PHYTANTRIOL UND P-HYDROXYACETOPHENONE
COMPOSITIONS TOPIQUES DE PHYTANTRIOLE ET P-HYDROXYACETOPHENONE

(30) Priority: 16.11.2017 EP 17202148
(43) Date of publication of application: 23.09.2020
(73) Proprietor: DSM IP Assets B.V., 6221 BE Maastricht (NL)
(72) Inventor: MENDROK-EDINGER, Christine, 4303 Kaiseraugst (CH); RUDOLPH, Thomas, 4303 Kaiseraugst (CH); SCHLIFKE-POSCHALKO, Alexander, 4303 Kaiseraugst (CH)
(74) Representative: dsm-firmenich IP
(86) International application number: PCT/EP2018/080384
(87) International publication number: WO 2019/096634

(56) References cited:
- KR-A- 20090 130 905
- US-A1- 2005 131 077
- HSIEH SUNG-FEI ET AL: "Chemical constituents from Farfugium japonicum var. formosanum", NATURAL PRODUCT COMMUNICATIONS, NATURAL PRODUCT INC, US, vol. 7, no. 4, 1 January 2012 (2012-01-01), pages 435 - 440, XP009502870, ISSN: 1934-578X
- DATABASE GNPD [online] MINTEL; 1 October 2015 (2015-10-01), "B-Complex + Biotin Thickening Shampoo", XP002777400, Database accession no. 3477243
- JI-YOUNG KIM: "Chemical composition and anti-inflammatory effects of essential oil from Farfugium japonicum flower", JOURANL OF OLEO SCIENCES, 31 December 2008 (2008-12-31), pages 623 - 628, XP055545568
- MEI-LING FENG ET AL: "Chemical constituents in fruits of Lycium barbarum", CHINESE TRADITIONAL AND HERBAL DRUGS, vol. 44, no. 3, 31 December 2013 (2013-12-31), pages 265 - 268, XP055545633, DOI: 10.7501/j.issn.0253-2670.2013.03.005

## Description

The present invention relates to topical compositions comprising phytantriol and hydroxyacetophenone wherein the ratio (w/w) of phytantriol to hydroxyacetophenone is > 1 as well as the use of phytantriol and hydroxyacetophenone for the prevention and/or treatment of ailments associated with Propionibacterium acnes.

Acne is taken to mean a skin disorder which is evident in inflamed papules, pustules or nodules, caused by increased talc production and impaired keratinization of the skin. The inflammation may be associated with reddening, swelling and pressure pain. Besides genetic predisposition, possible causes of acne formation can be androgens, comedogenic substances (for example in cosmetics), smoking, stress or excessive colonization of the skin by bacteria. Acne is in particular triggered by the microorganism Propionibacterium acnes (P. acnes), which is a bacterium which usually colonizes the skin and lives on sebum. Acne may arise, for example, if the number of these bacteria is increased. The presence of bacteria in the follicles results in inflammation reactions, which is evident in the form of red nodules or pustules. The production of free fatty acids by the bacteria furthermore promotes the inflammation reaction in the follicle. While many approaches to acne treatment have been reported, some have advocated use of a systemic or topical agent to address the overgrowth of Propionibacterium acnes.

Today, systemic therapy of acne often requires prescription antibiotics, such as erythromycin, tetracyclines, and clindamycin. In recent times however, physicians have become reluctant to over-prescribe antibiotics because resistance may be developed by not only acne-causing bacteria but other bacteria which are the causative agents of other more serious diseases. Furthermore, systemic administration may cause systemic side effects, as relatively high levels of the drug must circulate throughout the entire body.

Topical antibiotics which have been utilized to attempt to inhibit the overgrowth of P. acnes are clindamycin, erythromycin, tetracycline, and metronidazole. Each of these topical antibiotics reportedly cause side effects and widespread use also contributes to the risk of bacterial resistance.

Thus, there is an ongoing need for non-antibiotic alternatives, which can help to control the growth of P. acnes on the skin and are thus suitable for the treatment of any ailments associated with an overpopulation of P. acnes on the skin.

Surprisingly it has now been found that the combination of phytantriol and hydroxyacetophenone exhibits a synergistic antimicrobial activity against P. acnes. Thus, the combination can effectively be used to control the P. acnes on the skin and/ or the scalp and thus overcome the adverse effects resulting from a P. acnes overpopulation on the skin and/ or scalp such the treatment of inflammation triggered by P. acnes, red nodules or pustules.

Thus, the invention relates to the use of a combination of phytantriol and hydroxyacetophenone as antimicrobial agent against P. acnes.

It is well understood in all embodiments according to the present invention that phytantriol and hydroxyacetophenone are applied topically.

Topical compositions comprising the combination of phytantriol and hydroxyacetophenone, wherein the amount of phytantriol is higher than the amount of hydroxyacetophenone are still novel. This combination is furthermore particular effective for the inhibiting the growth of P. acnes.

Thus, the present invention also relates to topical compositions comprising phytantriol and hydroxyacetophenone, wherein the weight-ratio (ratio (w/w)) of phytantriol to hydroxyacetophenone is >1, according to the claims.

In a further embodiment, the invention relates to a method for killing and/ or inhibiting P. acnes, in particular on skin, said method comprising contacting said P. acnes with a combination of phytantriol and hydroxyacetophenone.

Due to the antimicrobial activity against P. acnes the combination of phytantriol and hydroxyacetophenone is further suitable for the treatment of any adverse skin condition associated with an overpopulation of P. acnes by maintaining skin homeostasis and/ or improving the health of the skin microbiome.

Thus, the invention also relates to a method of treating the skin and/ or the scalp, said method comprising the steps of contacting the skin and/ or scalp with a topical composition comprising phytantriol and hydroxyacetophenone for the treatment, prevention and/or prophylaxis of acne as well as for maintaining skin homeostasis and/ or skin microbiome balance.

In a further embodiment, the present invention relates to the use of a topical composition comprising phytantriol and hydroxyacetophenone for the treatment, prevention and/or prophylaxis of acne as well as for maintaining skin homeostasis and/ or skin microbiome balance.

Another suitable use according to the present invention is the treatment, prevention and/or prophylaxis of acne as the combination is also highly effective in killing/ inhibiting the growth of Propionibacterium acnes (P. acnes).

Phytantriol [CAS: 74563-64-7] is a colourless to light yellow, viscous liquid with the chemical name 3,7,11,15-tetramethyl-hexadecane-1,2,3-triol. Phytantriol is e.g. commercially available at DSM Nutritional Products Ltd, Kaiseraugst.

P-hydroxyacetophenone [CAS 99-93-4] is also called 1-(4-hydroxyphenyl)-ethanone and is e.g. commercially available at Symrise as SymSave^{®} H.

The term "antimicrobial activity" (or "antimicrobial effect") as used herein means a capability of killing and/or inhibiting the growth of P. acnes.

In all embodiments of the present invention the topical compositions preferably comprise phytantriol in an amount selected in the range of about 0.005 to 5 wt.-%, more preferably in the range of about 0.01 to 3 wt.-% and most preferably in the range of 0.025 to 2 wt.-%, such as in an amount of 0.04 to 1.5 wt.-% and particularly advantageous in an amount of 0.04 to 1 wt.-%, based on the total weight of the composition.

In all embodiments of the present invention the topical compositions preferably comprise hydroxyacetophenone in an amount selected in the range of 0.01 to 2 wt.-%, preferably in the range of 0.05 to 1.5 wt.-%, most preferably in the range of 0.1 to 1 wt.-%, based on the total weight of the composition.

To make use of the anti-microbial activity against P. acnes of the combination of phytantriol and hydroxyacetophenone, it can be used in a multiplicity of formulations or applications, such as, for example, cosmetic or pharmaceutical compositions, medicinal products or household products.

The use according to the invention of the combination of phytantriol and hydroxyacetophenone can take place both in the cosmetic sense as well as in the pharmaceutical sense. A pharmaceutical application is conceivable, for example, in the case of anti-acne compositions. In all embodiments of the present invention, the use is however preferably cosmetic (non-therapeutic) such as for healthy skin homeostasis and/ or for maintaining skin microbiome balance.

The topical compositions according to the present invention are preferably cosmetic or pharmaceutical compositions which are topically applied to mammalian keratinous tissue such as in particular to human skin or the human scalp and hair.

The term "cosmetic composition" as used in the present application refers to cosmetic compositions as defined under the heading "Kosmetika" in Römpp Lexikon Chemie, 10th edition 1997, Georg Thieme Verlag Stuttgart, New York as well as to cosmetic compositions as disclosed in A. Domsch, "Cosmetic Compositions", Verlag für chemische Industrie (ed. H. Ziolkowsky), 4th edition, 1992.

The cosmetic or pharmaceutical compositions according to the present invention preferably further comprise a physiologically acceptable medium, that is to say a medium compatible with keratinous substances, such as the skin, mucosa, and keratinous fibers. Preferably, the physiologically acceptable medium is a cosmetically or pharmaceutically acceptable carrier.

The term cosmetically or pharmaceutically acceptable carrier refers to all carriers and/or excipients and/ or diluents conventionally used in cosmetic compositions.

The topical compositions according to the present invention are generally prepared by admixing phytantriol and hydroxyacetophenone in the amounts indicated herein with a suitable carrier.

The exact amount of carrier will depend upon the actual level of phytantriol and hydroxyacetophenone and any other optional ingredients that one of ordinary skill in the art would classify as distinct from the carrier (e.g., other active ingredients).

In an advantageous embodiment, the cosmetic or pharmaceutical compositions according to the present invention comprise from about 50% to about 99%, preferably from about 60% to about 98%, more preferably from about 70% to about 98%, such as in particular from about 80% to about 95% of a carrier, based on the total weight of the cosmetic composition.

In a particular advantageous embodiment, the carrier consists furthermore of at least 40 wt.-%, more preferably of at least 50 wt.-%, most preferably of at least 55 wt.-% of water, such as in particular of about 55 to about 90 wt.-% of water.

The compositions of the invention (including the carrier) may comprise conventional adjuvants and additives, such as preservatives/antioxidants, fatty substances/oils, organic solvents, silicones, thickeners, softeners, emulsifiers, antifoaming agents, aesthetic components such as fragrances, surfactants, fillers, anionic, cationic, nonionic or amphoteric polymers or mixtures thereof, propellants, acidifying or basifying agents, dyes, colorings/colorants, abrasives, absorbents, chelating agents and/ or sequestering agents, essential oils, skin sensates, astringents, pigments or any other ingredients usually formulated into such compositions.

In accordance with the present invention, the compositions according to the invention may also comprise further cosmetically active ingredients conventionally used in cosmetic or pharmaceutical compositions. Exemplary active ingredients encompass skin lightening agents; UV-filters, agents for the treatment of hyperpigmentation; agents for the prevention or reduction of inflammation; firming, moisturizing, soothing, and/ or energizing agents as well as agents to improve elasticity and skin barrier.

Examples of cosmetic excipients, diluents, adjuvants, additives as well as active ingredients commonly used in the skin care industry which are suitable for use in the cosmetic compositions of the present invention are for example described in the International Cosmetic Ingredient Dictionary & Handbook by Personal Care Product Council (http://www.personalcarecouncil.org/), accessible by the online INFO BASE (http://online.personalcarecouncil.org/jsp/Home.jsp), without being limited thereto.

The necessary amounts of the active ingredients as well as the excipients, diluents, adjuvants, additives etc. can, based on the desired product form and application, easily be determined by the skilled person. The additional ingredients can either be added to the oily phase, the aqueous phase or separately as deemed appropriate.

The cosmetically active ingredients useful herein can in some instances provide more than one benefit or operate via more than one mode of action.

Of course, one skilled in this art will take care to select the above mentioned optional additional ingredients, adjuvants, diluents and additives and/or their amounts such that the advantageous properties intrinsically associated with the combination in accordance with the invention are not, or not substantially, detrimentally affected by the envisaged addition or additions.

Preferably, the cosmetic or pharmaceutical compositions according to the invention are in the form of a suspension or dispersion in solvents or fatty substances, or alternatively in the form of an emulsion or micro emulsion (in particular of O/W- or W/O-type), PIT-emulsion, nano emulsion, multiple emulsion (e. g. O/W/O- or W/O/W-type), pickering emulsion, hydrogel, lipogel, one- or multiphase solution or vesicular dispersion.

The cosmetic or pharmaceutical compositions in accordance with the invention can be in the form of a liquid, lotion, a thickened lotion, a gel, a cream, a milk, an ointment or a paste.

The cosmetic or pharmaceutical compositions according to the invention have a pH in the range of 3-10, preferably in the range of pH of 3-8, most preferred in the range of pH 3-7.5. The pH is adjusted by methods known to a person skilled in the art, e.g. by using an acid such as a hydroxy acid including glycolic acid, lactic acid, malic acid, citric acid and tartaric acid or a base such as e.g. sodium or potassium hydroxide or ammonium hydroxide as well as mixtures thereof.

Preferably, in the compositions according to the invention the acid, if present, is used in an amount of at least 0.0001 wt.-%, such as e.g. in an amount of 0.01-1 wt.-%, in particular in an amount of 0.01 to 0.5 wt.-%.

The cosmetic compositions according to the present invention advantageously comprise an additional preservative. Particular suitable preservatives in all embodiments of the present invention are benzoic acid, sodium benzoate, sorbic acid, potasssium sorbate, dehydroacetic acid, alcohol, alcohol denat.; as well as mixtures thereof. When present, the preservative is preferably used in an amount of 0.01 to 2 wt.-%, more preferably in an amount of 0.05 to 1.5 wt.-%, most preferably in an amount of 0.1 to 1.0 wt.-%, based on the total weight of the composition.

In another preferred embodiment, the compositions according to the present invention may comprise an additional amount of a preservation booster such as hydroxyacetophenone, caprylyl glycol, pentylene glycol, 1,2 hexanediol, decylene glycol, monoglycerides such as glyceryl laurate, propylene glycol caprylate, propylene glycol heptanoate as well as mixtures thereof.

When present, the preservation booster is preferably used in an amount of 0.01 to 2 wt.-%, more preferably in an amount of 0.05 to 1.5 wt.-%, most preferably in an amount of 0.1 to 1.0 wt.-%, based on the total weight of the composition.

The cosmetic compositions according to the present invention are in particular skin care preparations, functional preparations and/ or hair care preparations such as most in particularly skin or hair care preparations.

Examples of skin care preparations are, in particular, light protective preparations (sunscreen preparations), anti-ageing preparations, preparations for the treatment of photo-ageing, body oils, body lotions, body gels, treatment creams, skin protection ointments, moisturizing preparations such as moisturizing gels or moisturizing sprays, face and/or body moisturizers, as well as skin lightening preparations.

Examples of functional preparations are cosmetic compositions containing active ingredients such as hormone preparations, vitamin preparations, vegetable extract preparations, anti-ageing preparations, and/or antimicrobial (antibacterial or antifungal) preparations without being limited thereto.

Examples hair care preparations which are suitable according to the invention and which may be mentioned are shampoos, hair conditioners (also referred to as hair rinses), hairdressing compositions, hair tonics, hair regenerating compositions, hair lotions, water wave lotions, hair sprays, hair creams, hair gels, hair oils, hair pomades or hair brilliantines. Accordingly, these are always preparations which are applied to the hair and the scalp for a shorter or longer time depending on the actual purpose for which they are used.

If the hair care preparations according to the invention are supplied as shampoos, these can be clear liquids, opaque liquids (with pearly luster effect), in cream form, gel-like or else in powder form or in tablet form, and as aerosols. The surfactant raw materials on which these shampoos are based can be anionic, cationic, nonionic and amphoteric in nature and also be present in combinations of these substances.

Examples of anionic surfactants suitable for the incorporation into the shampoo preparations according to the present invention are C₁₀₋₂₀ alkyl- and alkylenecarboxylates, alkyl ether carboxylates, fatty alcohol sulfates, fatty alcohol ether sulfates, alkylolamide sulfates and sulfonates, fatty acid alkylolamide polyglycol ether sulfates, alkanesulfonates and hydroxyalkanesulfonates, olefinsulfonates, acyl esters of isothionates, alpha-sulfo fatty acid esters, alkylbenzenesulfonates, alkylphenol glycol ether sulfonates, sulfosuccinates, sulfosuccinic monoesters and diesters, fatty alcohol ether phosphates, protein-fatty acid condensation products, alkyl monoglyceride sulfates and sulfonates, alkyl glyceride ether sulfonates, fatty acid methyltaurides, fatty acid sarcosinates, and sulforicinoleates. These compounds and their mixtures are used in the form of their salts which are soluble in water or dispersible in water, for example the sodium, potassium, magnesium, ammonium, mono-, di- and triethanolammonium and analogous alkylanunonium salts.

Examples of suitable cationic surfactants are quaternary ammonium salts such as di(C₁₀₋-C₂₄alkyl)dimethylammonium chloride or bromide, preferably di (C₁₂-C₁₈alkyl)-dimethylammonium chloride or bromide; C₁₀-C₂₄-alkyldimethylethylammonium chloride or bromide; C₁₀-C₂₄-alkyltrimethylammonium chloride or bromide, preferably cetyltrimethylammonium chloride or bromide and C₂₀-C₂₄-alkyltrimethylammonium chloride or bromide; C₁₀-C₂₄4 -alkyldimethylbenzylammonium chloride or bromide, preferably C₁₂-C₁₈-alkyldime methylbenzylammoniumchloride; N-(C₁₂-C₁₈-alkyl)pyridinium chloride or bromide, preferably N- (C₁₂-C₁₆-alkyl)pyridinium chloride or bromide; N-(C₁₂-C₁₈-alkyl)isoquinolinium chloride, bromide or monoalkyl sulfate; N-(C₁₂-C₁₈-alkyloylcolaminoformylmethyl)pyridinium chloride; N-(C₁₂-C₁₈-alkyl)-N-methylmorpholinium chloride, bromide or monoalkyl sulfate; N-(C₁₂-C₁₈-alkyl)-N-ethylmorpholinium chloride, bromide or monoalkyl sulfate; C₁₆-C₁₈-alkylpentaoxethylammonium chloride; isobutylphenoxyethoxyethyldimethyl-benzylammonium chloride; salts of N,N-diethylaminoethylstearylamide and oleylamide with hydrochloric acid, acetic acid, lactic acid, citric acid, phosphoric acid; N-acylamidoethyl-N,N-diethyl-N-methylammonium chloride, bromide or monoalkylsulfate and N-acylaminoethyl-N,N-diethyl-N-benzylammonium chloride, bromide or monoalkyl sulfate, where acyl is preferably stearyl or oleyl.

Examples of suitable nonionic surfactants which can be used as detergent substances are fatty alcohol ethoxylates (alkylpolyethylene glycols); alkylphenol polyethylene glycols; alkyl mercaptan polyethylene glycols; fattyamine ethoxylates (alkylaminopolyethylene glycols); fatty acid ethoxylates (acylpolyethylene glycols); polypropylene glycol ethoxylates (Pluronic); fatty acid alkylolamides (fatty acid amide polyethylene glycols); sucrose esters; sorbitol esters and polyglycol ether.

Examples of amphoteric surfactants which can be added to the shampoos are N-(C₁₂-C₁₈-alkyl)-.beta.-aminopropionates and N-(C₁₂-C₁₈-alkyl)-.beta.-iminodipropionates as alkali metal and mono-, di- and trialkylammonium salts; N-acylamidoalkyl-N,N-dimethylacetobetaine, preferably N-(C₈-C₁₈-acyl)amidopropyl-N, N-dimethylacetobetaine; C₁₂-C₁₈-alkyldimethylsulfopropylbetaine; amphoteric surfactants based on imidazoline (commercial name: Miranol^{®}, Steinapon^{®}), preferably the sodium salt of 1-(β-carboxymethyloxyethyl)-1-(carboxymethyl)-2-laurylimidazolinium; amine oxide, for example C₁₂-C₁₈-alkyldimethylamine oxide, fatty acid amidoalkyldimethylamine oxide.

The hair care preparations according to the invention can additionally contain further additives customary in hair care such as for example perfumes, colorants, also those which simultaneously dye or tint the hair, solvents, opacifying agents and pearly luster agents, for example esters of fatty acids with polyols, magnesium and zinc salts of fatty acids, dispersions based on copolymers, thickening agents such as sodium, potassium and ammonium chloride, sodium sulfate, fatty acid alkylolamides, cellulose derivatives, natural rubbers, also plant extracts, protein derivatives such as gelatin, collagen hydrolysates, polypeptides with a natural or synthetic basis, egg yolk, lecithin, lanolin and lanolin derivatives, fats, oils, fatty alcohols, silicones, deodorizing agents, substances with antimicrobial activity, substances with antiseborrhoeic activity, substances with keratolytic and keratoplastic effect, such as, for example, sulfur, salicylic acid and enzymes as well as further anti-dandruff agents such as olamine, climbazol, zink pyrithion, ketoconazole, salicylic acid, sulfur, tar preparations, derivatives of undecenic acid, extracts of nettel, rosmary, cottonwood, birch, walnut, willow bark and/ or arnica.

The topical cosmetic compositions according to the present invention are advantageously O/W emulsions, W/O emulsions and/ or gels such as shower gels.

Furthermore, the topical compositions in the form of O/W emulsions, W/O emulsions and/ or gels according to the present invention are skin care preparation intended for the treatment of acne or for maintaining a healthy skin homeostasis and/ or for maintaining skin microbiome balance.

O/W emulsions according to the present invention advantageously comprise (i) phytantriol in an amount selected in the range of 0.005 to 3 wt.-%, more preferably in the range of about 0.01 to 2 wt.-%, most preferably in the range of 0.025 to 1.5 wt.-% and particularly advantageous in an amount of 0.04 to 1 wt.-%, based on the total weight of the composition.
(ii) p-hydroxacetophenone in an amount selected in the range of about 0.01 wt.-% to 2 wt.-%, more preferably in the range of 0.05 to 1.5 wt.-%, most preferably in the range of 0.1 to 1 wt.-%, and particularly advantageous in the range of 0.15 to 1 wt.-% based on the total weight of the composition, (iii) water and (iv) at least one O/W- or Si/W-emulsifier selected from the list of glycerylstearatcitrate, glycerylstearate (self emulsifying), stearic acid, salts of stearic acid, polyglyceryl-3-methylglycosedistearate, ceteareth-20, steareth-2, steareth-12, PEG-40 stearate, phosphate esters and the salts thereof such as cetyl phosphate (Amphisol^{®} A), diethanolamine cetyl phosphate (Amphisol^{®} DEA), potassium cetyl phosphate (Amphisol^{®} K), sodiumcetearylsulfat, sodium glyceryl oleate phosphate, hydrogenated vegetable glycerides phosphate, sorbitan oleate, sorbitan sesquioleate, sorbitan isostearate, sorbitan trioleate, lauryl glucoside, decyl glucoside, sodium stearoyl glutamate, sucrose polystearate and Hydrated Polyisobuten as well as mixtures thereof. Also, one or more synthetic polymers may be used as an emulsifier such as for example, PVP eicosene copolymer, acrylates/C10-3o alkyl acrylate crosspolymer, acrylates/steareth-20 methacrylate copolymer, PEG-22/dodecyl glycol copolymer, PEG-45/dodecyl glycol copolymer, and mixtures thereof. In a particular preferred embodiment the O/W-emulsifier is selected from the group of cetyl phosphates such as in particular potassium cetyl phosphate (commercially available as Amphisol^{®} K), glyceryl stearate (and) PEG 100 stearate (commercially available as Arlacel^{®} 165) and/ or polyalkylenglycolether such as in particular laureth-35 (lauryl alcohol with 35 EO units; commercially available as Brij^{®} 35).The at least one O/W emulsifier is preferably used in an amount of about 0.001 to 10 wt.-%, more preferably in an amount of 0.1 to 7 wt.-% with respect to the total weigh of the composition. Additionally, the cosmetic composition in the form of a O/W emulsion contains advantageously at least one co-emulsifier selected from the list of alkyl alcohols such as Cetyl Alcohol (Lorol C16, Lanette 16) Cetearyl Alcohol (Lanette^{®} O), Stearyl Alcohol (Lanette^{®} 18), Behenyl Alcohol (Lanette^{®} 22), Glyceryl Monostearate, Glyceryl Myristate (Estol^{®} 3650), Hydrogenated Coco- Glycerides (Lipocire Na10) without being limited to this and mixtures thereof.

W/O emulsions according to the present invention advantageously comprise (i) phytantriol in an amount selected in the range of 0.005 to 3 wt.-%, more preferably in the range of about 0.01 to 2 wt.-%, most preferably in the range of 0.025 to 1.5 wt.-% and particularly advantageous in an amount of 0.04 to 1 wt.-%, based on the total weight of the composition.
(ii) p-hydroxacetophenone in an amount selected in the range of about 0.01 wt.-% to 2 wt.-%, more preferably in the range of 0.05 to 1.5 wt.-%, most preferably in the range of 0.1 to 1 wt.-%, and particularly advantageous in the range of 0.15 to 1 wt.-% based on the total weight of the composition, based on the total weight of the composition, (iii) water and (iv) at least one W/O- or W/Si-emulsifier selected from the list of polyglyceryl-2-dipolyhydroxystearat, PEG-30 dipolyhydroxystearat, cetyl dimethicone copolyol, polyglyceryl-3 diisostearate polyglycerol esters of oleic/isostearic acid, polyglyceryl-6 hexaricinolate, polyglyceryl-4-oleate, polygylceryl-4 oleate/PEG-8 propylene glycol cocoate, magnesium stearate, sodium stearate, potassium laurate, potassium ricinoleate, sodium cocoate, sodium tallowate, potassium castorate, sodium oleate, and mixtures thereof. Further suitable W/Si-emulsifiers are Lauryl Polyglyceryl-3 Polydimethylsiloxyethyl Dimethicone and/or PEG-9 Polydimethylsiloxyethyl Dimethicone and/or Cetyl PEG/PPG-10/1 Dimethicone and/or PEG-12 Dimethicone Crosspolymer and/or PEG/PPG-18/18 Dimethicone. The at least one W/O emulsifier is preferably used in an amount of about 0.001 to 10 wt.-%, more preferably in an amount of 0.2 to 7 wt.-% with respect to the total weigh of the composition.

Gel preparations according to the present invention advantageously comprise (i) phytantriol in an amount selected in the range of 0.005 to 3 wt.-%, more preferably in the range of about 0.01 to 2 wt.-%, most preferably in the range of 0.025 to 1.5 wt.-% and particularly advantageous in an amount of 0.04 to 1 wt.-%, based on the total weight of the composition.
(ii) p-hydroxacetophenone in an amount selected in the range of about 0.01 wt.-% to 2 wt.-%, more preferably in the range of 0.05 to 1.5 wt.-%, most preferably in the range of 0.1 to 1 wt.-%, and particularly advantageous in the range of 0.15 to 1 wt.-%, based on the total weight of the composition, based on the total weight of the composition, (iii) water and (iv) at least one water soluble thickener. Such water-soluble thickeners are well known to a person skilled in the art and are e.g. listed in the "Handbook of Water soluble gums and resins" by Robert L. Davidson (Mc Graw Hill Book Company (1980)). Particularly suitable water soluble thickeners are selected from the group consisting of polyacrylic acids (e.g. commercially available under the tradename Carbomer or Carbopol^{®}), homopolymers of 2-Acrylamido-2-methylpropansulfonic acid (e.g. commercially available as Rheothik^{®}11-80), acrylate copolymers (e.g. commercially available under the tradename Pemulen^{®} or Aculyn^{®} 33), branched Poly(methacryloyloxyethyltrimethylammoniumchlorid) (INCI-name Polyquaternium-37), non-modified guar gums (e.g. commercially available under the tradename Jaguar), starch or derivatives thereof and/ or hydroxyalkylcellulosen. Preferably the water-soluble thickener is used in an amount of about 0.001 to 10 wt.-%, more preferably in an amount of 0.2 to 7 wt.-%, based on the total weigh of the composition.

The following examples are provided to further illustrate the compositions and effects of the present invention. These examples are illustrative only and are not intended to limit the scope of the invention in any way.

### Example 1: Antimicrobial efficacy against Propionibacterium acnes

The antimicrobial efficacy is assessed in analogy to the regulatory challenge test method (NF EN ISO11930). Thus, solutions of the respective active(s) in ethanol are prepared and further dissolved in physiological serum with 0.85 wt.-% NaCl in the concentrations as outlined in table 1 under sterile conditions. Samples containing phytantriol (Phytantriol from DSM Nutritional Products Ltd) were solubilized in physiological serum supplemented with 10 wt.-% ethanol, samples containing p-hydroxyacetophenone (p-HAP; SymSave^{®} H from Symrise) only were solubilized in physiological serum supplemented with 1 wt.-% ethanol. The respective solutions of the active(s) were deposed in 96-deep well plates (1.6 ml/well). The wells are contaminated with P. acnes at 2.5*10⁵ to 5.6*10⁵ cfu/ml to obtain the initial contamination as outlined in table 1. After the contamination, each well was thoroughly mixed to ensure a homogeneous distribution of P. acnes. Then each plate was incubated at 22°C for 24h. The counting of the (remaining) population is carried out 24h after contamination.

**Table 1: Results**

| Test solution | Time [h] | P. acnes colony count [cfu/ml] | Δ* | Log reduction |
|---|---|---|---|---|
| 0.2 wt.-% phytantriol | 0 | 310000 | | |
| | 24 | 28000 | -90.97% | -1.044 |
| 0.1 wt.-% p-HAP | 0 | 310000 | | |
| | 24 | 260000 | -16.13% | -0.076 |
| 0.2 wt.-% phytantriol | 0 | 310000 | | |
| 0.1 wt.-% p-HAP | 24 | 530 | -99.83% | -2.767 |
| 0.3 wt.-% phytantriol | 0 | 310000 | | |
| | 24 | 3300 | -98.94% | -1.973 |
| 0.3 wt.-% p-HAP | 0 | 310000 | | |
| | 24 | 5500 | -98.23% | -1.751 |
| 0.1 wt.-% phytantriol | 0 | 310000 | | |
| 0.2 wt.-% p-HAP | 24 | 3000 | -99.03% | -2.041 |

| | | | | |
|---|---|---|---|---|
| *Δ = -({microorganism count t=0} - {microorganism count t = 24h}/ {microorganism count t=0})*100 | | | | |

As can be seen in the table above the combination of phytantriol and hydroxyacetophenone shows a synergistic effect against P. acnes. The combination of a ratio of phytantriol : hydroxyacetophenone of 2:1 (1.5:1 to 3:1) performs best as can be retrieved from the log reduction.

### Example 4: W/O Cream

| *Ingredients* | *INCI* | *wt. %* |
|---|---|---|
| Cremophor WO-7 | PEG-7 Hydrogenated Castor Oil | 2.50 |
| Elfacos ST-9 | PEG-45/Dodecyl Glycol Copolymer | 2.00 |
| Cirebelle 303 | Synthetic Wax | 5.00 |
| Cirebelle 109L | Synthetic Wax | 7.20 |
| Miglyol 818 | Caprylic/Capric/Linoleic Triglyceride | 5.00 |
| Eutanol G | Octyldodecanol | 7.50 |
| Cetiol OE | Dicaprylyl Ether | 9.20 |
| Deionised Water | Aqua | Ad 100 |
| Glycerin | Glycerin | 5.00 |
| Propylene Glycol | Propylene Glycol | 2.00 |
| Euxyl PE 9010 | Phenoxyethanol and Ethylhexylglycerin | 0.80 |
| Phytantriol | Phytantriol | 0.3 |
| p-Hydroxyacetophenone | Hydroxyacetophenone | 0.1 |

## Claims

1. A topical composition comprising phytantriol and p-hydroxyacetophenone, wherein the ratio (w/w) of phytantriol to p-hydroxyacetophenone is 1.5:1 to 3:1.

2. The topical composition according to claim 1, wherein the amount of phytantriol in an amount selected in the range of about 0.005 to 5 wt.-%, preferably in the range of about 0.01 to 3 wt.-%, most preferably in the range of 0.025 to 2 wt.-%, based on the total weight of the composition.

3. The topical composition according to claim 1 or 2, wherein the amount of p-hydroxyacetophenone is selected in the range of about 0.01 to 2 wt.-%, preferably in the range of 0.05 to 1.5 wt.-%, most preferably in the range of 0.1 to 1 wt.-%, based on the total weight of the composition.

4. The topical composition according to any one of claims 1 to 3 wherein the composition is a cosmetic or pharmaceutical composition.

5. The topical composition according to claim 4, wherein the composition is a shampoo preparation, an O/W emulsions, a W/O emulsion or a gel.

6. The topical composition according to any one of claim 1 or 5, wherein the composition furthermore comprises water and at least one agent selected from the group consisting of surfactants, emulsifiers, thickeners and oils.

7. The topical composition according to any one of claims 1 to 6 for maintaining a healthy skin homeostasis and/ or for maintaining skin microbiome balance.

8. The topical composition according to any one of claims 1 to 6 for use in the treatment or prevention of Propionibacterium acnes induced diseases and/ or disorders.

9. The topical composition according to claim 8 for use in the treatment or prevention of acne.

10. A combination of phytantriol and p-hydroxyacetophenone in a ratio (w/w) of 1.5:1 to 3:1 for use in killing and/or inhibiting Propionibacterium acnes on skin, by contacting said Propionibacterium acnes with said combination of phytantriol and hydroxyacetophenone.

## Patentansprüche

1. Topische Zusammensetzung, umfassend Phytantriol und p-Hydroxyacetophenon, wobei das Verhältnis (w/w) von Phytantriol zu p-Hydroxyacetophenon 1,5:1 bis 3:1 beträgt.

2. Topische Zusammensetzung nach Anspruch 1, wobei die Menge von Phytantriol eine Menge ist, die im Bereich von etwa 0,005 bis 5 Gew.-%, vorzugsweise im Bereich von etwa 0,01 bis 3 Gew.-%, ganz besonders bevorzugt im Bereich von 0,025 bis 2 Gew.-%, bezogen auf das Gesamtgewicht der Zusammensetzung, ausgewählt ist.

3. Topische Zusammensetzung nach Anspruch 1 oder 2, wobei die Menge von p-Hydroxyacetophenon im Bereich von etwa 0,01 bis 2 Gew.-%, vorzugsweise im Bereich von 0,05 bis 1,5 Gew.-%, ganz besonders bevorzugt im Bereich von 0,1 bis 1 Gew.-%, bezogen auf das Gesamtgewicht der Zusammensetzung, ausgewählt ist.

4. Topische Zusammensetzung nach einem der Ansprüche 1 bis 3, wobei es sich bei der Zusammensetzung um eine kosmetische oder pharmazeutische Zusammensetzung handelt.

5. Topische Zusammensetzung nach Anspruch 4, wobei es sich bei der Zusammensetzung um eine Shampoozubereitung, eine O/W-Emulsion, eine W/O-Emulsion oder ein Gel handelt.

6. Topische Zusammensetzung nach einem der Ansprüche 1 oder 5, wobei die Zusammensetzung weiterhin Wasser und mindestens ein Mittel, das aus der Gruppe bestehend aus Tensiden, Emulgatoren, Verdickungsmitteln und Ölen ausgewählt ist, umfasst.

7. Topische Zusammensetzung nach einem der Ansprüche 1 bis 6 zur Aufrechterhaltung einer gesunden Hauthomöostase und/oder zur Aufrechterhaltung der Hautmikrobiombalance.

8. Topische Zusammensetzung nach einem der Ansprüche 1 bis 6 zur Verwendung bei der Behandlung oder Prävention von durch Propionibacterium acnes induzierten Erkrankungen und/oder Störungen.

9. Topische Zusammensetzung nach Anspruch 8, wobei die Zusammensetzung zur Verwendung bei der Behandlung oder Prävention von Akne dient.

10. Kombination von Phytantriol und p-Hydroxyacetophenon in einem Verhältnis (w/w) von 1,5:1 bis 3:1 zur Verwendung bei der Abtötung und/oder Inhibierung von Propionibacterium acnes auf der Haut durch Inkontaktbringen des Propionibacterium acnes mit der Kombination von Phytantriol und p-Hydroxyacetophenon.

## Revendications

1. Composition topique comprenant du phytantriol et de la p-hydroxyacétophénone, le rapport (p/p) de phytantriol sur p-hydroxyacétophénone étant 1,5:1 à 3:1.

2. Composition topique selon la revendication 1, la quantité de phytantriol étant une quantité choisie dans la plage d'environ 0,005 à 5 % en poids, préférablement dans la plage d'environ 0,01 à 3 % en poids, le plus préférablement dans la plage de 0,025 à 2 % en poids, sur la base du poids total de la composition.

3. Composition topique selon la revendication 1 ou 2, la quantité de p-hydroxyacétophénone étant choisie dans la plage d'environ 0,01 à 2 % en poids, préférablement dans la plage de 0,05 à 1,5 % en poids, le plus préférablement dans la plage de 0,1 à 1 % en poids, sur la base du poids total de la composition.

4. Composition topique selon l'une quelconque des revendications 1 à 3, la composition étant une composition cosmétique ou pharmaceutique.

5. Composition topique selon la revendication 4, la composition étant une préparation de shampooing, une émulsion H/E, une émulsion E/H ou un gel.

6. Composition topique selon l'une quelconque des revendications 1 ou 5, la composition comprenant par ailleurs de l'eau et au moins un agent choisi dans le groupe constitué par des tensioactifs, des émulsifiants, des épaississants et des huiles.

7. Composition topique selon l'une quelconque des revendications 1 à 6 pour le maintien d'une homéostasie de la peau saine et/ou pour le maintien de l'équilibre du microbiome de la peau.

8. Composition topique selon l'une quelconque des revendications 1 à 6 pour une utilisation dans le traitement ou la prévention de maladies et/ou de troubles induit(e)s par Propionibacterium acnes.

9. Composition topique selon la revendication 8, ladite composition étant pour une utilisation dans le traitement ou la prévention de l'acné.

10. Combinaison de phytantriol et de p-hydroxyacétophénone en un rapport (p/p) de 1,5:1 à 3:1 pour une utilisation dans la destruction et/ou l'inhibition de Propionibacterium acnes sur la peau, par mise en contact dudit Propionibacterium acnes avec ladite composition de phytantriol et d'hydroxyacétophénone.
